Europäisches Patentamt

(19) European Patent Office

Office européen des brevets

(11) Publication number: 0 165 696
A2

## (12) EUROPEAN PATENT APPLICATION

(21) Application number: 85303283.7

(22) Date of filing: 09.05.85

(51) Int. Cl.⁴: A 61 K 47/00
A 61 L 15/06

(30) Priority: 10.05.84 US 608835

(43) Date of publication of application:
27.12.85 Bulletin 85/52

(84) Designated Contracting States:
AT BE CH DE FR IT LI LU NL SE

(71) Applicant: AMERICAN HOME PRODUCTS
CORPORATION
685, Third Avenue
New York, New York 10017(US)

(72) Inventor: Sarpotdar, Pramod Purushottam
13 Brenda Lane Audubon
Montgomery Pennsylvania 19403(US)

(72) Inventor: Gaskill, James Lawrence
500 East Lancaster Avenue St. Davids
Delaware Pennsylvania(US)

(72) Inventor: Giannini, Robert Peter
508 Meadowbrook Road East Norriton
Montgomery Pennsylvania(US)

(74) Representative: Wileman, David Francis et al,
c/o John Wyeth and Brother Limited Huntercombe Lane
South
Taplow Maidenhead Berkshire SL6 OPH(GB)

(54) Improved transdermal dosage form.

(57) The invention concerns an improved pharmaceutical composition and dosage form for the transdermal delivery of a therapeutic agent dispersed in a solvent system comprising a mixture of propylene glycol and glycerine in the ratio of about 1:1 to about 1:5 by weight of each other and if required such other pharmaceutically acceptable solvent or solvents necessary to achieve a solubility of at least 0.5 milligrams millilitre of the therapeutic agent in the solvent system, the amount of propylene glycol and glycerine being such as to enhance the transdermal penetration of the therapeutic agent.

Croydon Printing Company Ltd

CLAIMS FOR AUSTRIA

1.   A process for preparing a transdermal pharmaceutical
     composition which comprises bringing into
     association a transdermally effective therapeutic
     agent, propylene glycol, glycerine and if
     required such other solvent or solvents to achieve
     a solubility of at least about 0.5 milligrams per
     millilitre of the therapeutic agent in the mixture
     of solvents, the ratio of propylene glycol to
     glycerine being from about 1:1 to 1:5 by weight
     of each other and the amount of propylene glycol -
     glycerine mixture being such as to enhance the
     penetration of the drug.

2.   A process for preparing a pharmaceutical dosage
     form which comprises bringing into association
     a matrix or matrix forming agent, a transdermally
     effective therapeutic agent, propylene glycol,
     glycerine and additionally if required such
     other solvent or solvents to achieve a solubility
     of at least 0.5 milligrams per millilitre of
     the therapeutic agent in the mixture of solvents;
     the ratio of propylene glycol to glycerine being
     from about 1:1 to 1:5 by weight of each other
     and the amount of propylene glycol - glycerine
     mixture being such as to enhance the penetration
     of the drug, and if required forming  the matrix
     from the matrix forming agent at any suitable stage.

3.   A process as claimed in Claim 2 in which the
     matrix is a non-polymeric gel.

4.   A process as claimed in Claim 3 in which the
     non-polymeric gel comprises an alkali metal
     salt of a fatty acid.

5. A process as claimed in Claim 4 in which the non-polymeric matrix comprises a gelling agent selected from sodium stearate, sodium caprylate, sodium laurate, sodium myristate, sodium palmilate, sodium oleate, potassium caprylate, potassium laurate, potassium myristate, potassium palmitate, potassium oleate and potassium stearate.

6. A process as claimed in any one of Claims 1 to 5 in which the propylene glycol-glycerine mixture comprises 10 to 93 per cent by weight of the composition.

7. A process as claimed in any one of Claims 1 to 6 which the propylene glycol: glycerine ratio is about 35:65.

8. A process as claimed in any one of Claims 1 to 7 further comprising azone and/or ethanol.

9. A process as claimed in any one of Claims 1 to 8 wherein the therapeutic agent is soluble in the solvent system in an amount of at least 3 milligrams per millilitre of solvent.

10. A process as claimed in any one of Claims 1 to 9 wherein the therapeutic agent comprises 1 to 50 per cent of the composition.

This invention relates to transdermal compositions and dosage forms. More particularly, this invention relates to transdermal compositions and dosage forms in which the penetration of the active ingredient is enhanced by the presence of a combination of propylene glycol and glycerine in certain defined ratios, to transdermal dosage forms in which the active ingredient is dispersed in a polymeric matrix and to methods of preparing such compositions and dosage forms.

A number of different forms for transdermal release systems for pharmaceutical preparations are known in the prior art. Many of these take the form of adhesive bandages which are attached to the skin, have an outer impervious covering of foil or the like to contain the active ingredients in a chamber next to the skin and serve to hold the active ingredients in contact with the skin and to avoid mussing or soiling the clothing of the patient.

The active ingredient is either supported on the outer surface of a structurally sufficient support layer or dispersed through it. The active ingredient may be in solid form or may be suspended or dissolved in an organic or aqueous solvent. Typically, the structural support layer is a porous polymeric membrane. Particular polymers have been identified for use, such as the polyacrylates. In many such forms, the rate of absorption of the active ingredient into the patient's system is controlled by the porosity of the support layer. Various adjuvants, both hydrophilic and hydrophobic are known to be useful to increase the rate of release to the skin, or to reduce the rate of release to the skin and so control the duration of the sustained release to the skin.

In some known devices, a second semi-porous polymeric membrane is placed between the skin and the active ingredient. The porosity of the second layer controls the rate of release from the active ingredient support structure to the skin. This type of device is known as a membrane controlled rate of release device.

Some prior art devices use compounds which enhance the rate of penetration through the skin, such as dimethylsulfoxide and azone (1-dodecylazacyclopentan-2-one). Other devices are known which make use of a matrix made up of polyvinyl alcohol and polyvinylpyrrolidone which are water soluble and may be replaced with gum arabic, gum tragacanth, polyacrylic acid, polymethacrylic acid, polyvinyloxazolidone, polyvinylmorpholinone and polyvinyl-piperidone. These are self-supporting polymeric diffusion matrices which contain the active ingredient along with a polar plasticizer such as glycerine, which is a necessary component of the matrix and is used to make the matrix flexible and permit the matrix to have a good diffusional contact with the skin.

It is also known in the prior art to use silicone polymers as a matrix into which is mixed a hydrophilic solvent system containing an active ingredient and a hydrophobic solvent which enhances the dispersion and transport of the active ingredient (U.S. Patent 4,336,243). U.S. Patent 3,996,934 discloses a medical bandage comprising a backing member and a facing member having a reservoir containing an active ingredient, either as a distinct layer, or as a plurality of microcapsules distributed throughout the silicone polymer matrix and lists a large number of solvents including glycerine and ethanol but does not suggest the advantages of a combination of glycerine and propylene glycol as a solvent system.

Some of the prior art devices made use of propylene glycol as ingredients in a carrier composition. None suggest that the combined use of propylene glycol and glycerine in certain defined ratios enhance the transdermal penetration of the active ingredient. U.S. Patent 3,989,816 discloses a method for transdermally administering an active ingredient by dissolving the ingredient in a carrier containing azone. A long list of suitable inert carriers includes ethanol, propylene glycol and stearic acid. There is no suggestion of the advantages of propylene glycol and glycerine in combination or that propylene glycol or stearic acid enhance the penetration of the active ingredient through the skin. U.S. Patent 3,527,864 describes compositions for topical application to animal tissue and a method for enhancing penetration thereof. The penetration-enhancing ingredients are aliphatic sulfoxides. Propylene glycol, glycerine and ethanol individually are included with many other compounds in composition with the aliphatic sulfoxides. There is no teaching or suggestion that combinations of propylene glycol and glycerine in any ratio themselves enhance the penetration.

U.S. Patent 4,409,206 describes transdermal release systems for pharmaceutical preparations in which one of the elements is a hydrophilic substance which may be selected from a multi-member class which includes glycerine. The hydrophilic auxiliary substance, which is stated to contain glycerine among other compounds, is described as being present to regulate the release of the pharmaceutical. There is no suggestion that the rate of penetration of an active ingredient is enhanced by certain ratios of glycerine and propylene glycol used in combination.

U.S. Patent 4,307,717 describes a bandage containing an ingredient which has a substrate that is a stable matrix of synthetic and/or natural gums containing a liquid phase consisting of polyhydric alcohols, such as glycerine or propylene glycoland many other ingredients. The bandages are described for topical use and topical treatment. The patent does not suggest the use of glycerine and propylene glycol in combination in the ratios of the present invention enhance transdermal penetration of an active ingredient.

U.S. Patent 4,291,015 states that either propylene glycol or glycerine among others may be used as a polar plasticizer in a polymeric diffusion matrix, but does not teach or suggest that a combination of propylene glycol and glycerine in certain ratios enhances the rate of penetration of the active ingredient.

U.S. Patent 4,420,470 describes a transdermal device for isosorbide dinitrate and states that propylene glycol and many other alcohols may be used as an absorption promoter, similar to dimethylsulfoxide. There is no teaching or suggestion that enhanced rate of penetration is improved by particular combinations of propylene glycol and glycerine.

Similarly, patents nos. 3,952,099 and 4,274,420 describe both glycerine and propylene glycol as useful as carriers or electrically conductive materials in transdermal devices without teaching or suggesting combinations of the two in certain ratios which greatly enhance the rate of penetration of an active ingredient.

In one aspect this invention provides a pharmaceutical composition for the transdermal delivery of a therapeutic agent dispersed in a solvent system characterised in that the solvent system comprises a mixture of propylene glycol and glycerine in the ratio of about 1:1 to about 1:5 by weight of each other and if required such other pharmaceutically acceptable solvent or solvents necessary to achieve a solubility of at least 0.5 milligrams/millilitre of the therapeutic agent in the solvent system, the amount of propylene glycol and glycerine being such as to enhance the transdermal penetration of the therapeutic agent (active ingredient).

Preferred compositions according to this invention are those in which the solubility of the therapeutic agent in the solvent system is at least 3.0 milligrams per millilitre. Also preferred are such compositions in which the solvent system consists substantially of propylene glycol and glycerine in a ratio of about 1:1 to 1:4 by weight of each other. Such compositions may also contain a further penetration enhancer in an amount effective to further enhance transdermal penetration of the therapeutic agent. Also preferred are such compositions dispersed in a matrix. Although these matrices may include non-polymeric matrices and porous polymeric matrices as described below (and in some of the United States patents referred to above), the preferred matrix is a non-polymeric matrix formed from a gelling agent comprising a metal salt of a fatty acid. The sodium or potassium salt is preferred.

This invention further provides a method of enhancing the transdermal penetration of a therapeutic agent characterised in dispersing said therapeutic agent in a solvent system comprising of a mixture of propylene glycol and glycerine in a ratio of about 1:1 to 1:5 by weight of each other and, if required, including in such solvent system such other pharmaceutically acceptable solvent or solvents to achieve a solubility of at least 0.5 milligrams per millilitre of the therapeutic agent in the solvent system. The preferred embodiments of the described method are the same as described above for the pharmaceutical composition. Preferred therapeutic agents (active ingredients) for use in the pharmaceutical composition and method of enhancing transdermal delivery are those listed in Table A below.

This invention further provides a drug delivery system suitable for the transdermal administration of active ingredients comprising an impervious backing and a matrix attached thereto, suitable for attaching to the
skin, said matrix further comprising:

a) a gelling or a matrix forming agent,

b) a solvent system dispersed in said gelling or
matrix forming agent comprising a mixture of
propylene glycol and glycerine in a ratio of
1:1 to 1:5 by weight of each other and, if
required, such other pharmaceutically acceptable
solvent or solvents to achieve a solubility of
at least 0.5 milligrams per millilitre of the
therapeutic agent in the solvent system, and

c) a therapeutic agent dispersed in said solvent
system.

Preferred gelling agents for such a drug delivery system
are metal salts of fatty acids which provide a non-polymeric
matrix as described herein. Sodium, potassium salts are
particularly preferred. Sodium stearate is particularly
preferred. A preferred solvent system is a nonaqueous
solvent system consisting substantially of
propylene glycol and glycerine in a ratio of about 1:1
to 1:5 by weight of each other. As noted herein, such drug
delivery system may also include further penetration
enhancers.

A further embodiment of a drug delivery system of the
invention comprises an impervious backing and a matrix
attached thereto suitable for attaching to the skin, said
matrix further comprising:

a) from 3 to 50 percent by weight of a solid non-
polymeric gel;

b) from 10 to 93 percent by weight of a nonaqueous
liquid medium dispersed in said non-polymeric gel
comprising a mixture of propylene glycol and
glycerine in a ratio of 1:1 to 1:5 by weight;
and

c) from one to 50 percent by weight of an active ingredient dispersed in said liquid medium.

The invention further provides a pharmaceutical dosage form for transdermal administration of an active ingredient comprising a thin flexible non-polymeric matrix in planar form further comprising:

a) about 3 to 50 percent by weight of a metal salt of a fatty acid;

b) about 10 to 93 percent by weight of solvent medium comprising propylene glycol and glycerine in a ratio of about 1:1 to 1:4 by weight of each other; and

c) from 1 to 50 percent by weight of an active ingredient soluble in said solvent medium in an amount of at least 3 milligrams per millilitre.

Such pharmaceutical dosage form is particularly preferred for the ingredients guanabenz and 17-beta-estradiol. In such pharmaceutical dosage form the preferred salt of a fatty acid is sodium stearate, and the preferred amount thereof is about 6 percent by weight of the total weight. Also preferred is a pharmaceutical dosage form having an impervious backing material connected to one side of said matrix.

This invention further includes a process for preparing a transdermal pharmaceutical composition which comprises bringing into association a transdermally effective therapeutic agent, propylene glycol, glycerine and, if required, such other solvent or solvents to achieve a solubility of at least about 0.5 milligrams per millilitre of the therapeutic agent in the mixture of solvents, the ratio of propylene glycol to glycerine being from about 1:1 to 1:5 by weight of each other and the amount of propylene glycol/glycerine mixture being such as to enhance the penetration of the drug.

This invention also provides a process for preparing a pharmaceutical dosage form which comprises bringing into association a matrix or matrix forming agent, a transdermally effective therapeutic agent, propylene glycol, other solvent or solvents to achieve a solubility of at least 0.5 milligrams per millilitre of the therapeutic agent in the mixture of solvents; the ratio of propylene glycol to glycerine being from about 1:1 to 1:5 by weight of each other and the amount of propylene glycol - glycerine mixture being such as to enhance the penetration of the drug, and if required forming the matrix from the matrix forming agent at any suitable stage.

Preferred means of carrying out said processes will be apparent to the reader from the description and examples given below.

The invention also includes a patch suitable for transdermal administration of an active ingredient comprising a non-polymeric matrix containing from 10 to 93 percent by weight of a nonaqueous solvent medium comprising about 15 to 50 percent by weight of propylene glycol and about 50 to 85 percent by weight of glycerine and a therapeutically effective amount of an active ingredient dissolved in said solvent medium, wherein the propylene glycol and glycerine are in ratio of about 1:1 to 1:5 by weight of each other.

Preferred embodiments for such patches are described in the examples given below. Particularly preferred is such a transdermal patch in which the active ingredient is soluble in said solvent medium in the amount of at least 3 milligrams per millilitre. Preferred active ingredients for inclusion in the patches of the invention are guanabenz and 17-beta-estradiol.

The objects of the invention may further be achieved with a pharmaceutical dosage form for transdermal administration of an active ingredient which is dispersed in a non-polymeric matrix. The matrix may be comprised of an alkaline metal salt of a fatty acid preferably in the amount of from 3 to 50, desirably about six percent by weight of the total weight of matrix, and a solvent system and active ingredient, as well as fillers and extenders and the like which may be present. The active ingredient is dispersed in the solvent system which is dispersed in the matrix.

The preferred gelling agent for forming the matrix is sodium stearate, but other alkaline metal salts of fatty acids also may be used, such as sodium caprylate, sodium laurate, sodium myristate, sodium palmitate, sodium oleate, sodium stearate, potassium caprylate, potassium laurate, potassium myristate, potassium palmitate, potassium oleate, potassium stearate, and the corresponding salts of a fatty acid with calcium, magnesium, aluminium, zinc or other metals. The sodium and potassium salts are preferred.

The matrix may be polymeric providing it is compatible with the solvent system. The solvent system is a penetration enhancing material made up of propylene glycol and glycerine in a ratio about 1:1 to 1:5 by weight to each other. That is, the propylene glycol may be about 15 to 50 percent by weight of the combined weight with glycerine. The combination of propylene glycol and glycerine comprises about 10 to 93 weight percent of the total weight of the composition or dosage form, which includes an active ingredient, propylene glycol and glycerine, fillers and extenders, matrix or matrix forming agents, and the like. Propylene glycol in excess of fifty percent in the solvent system may cause the matrix to be brittle.

The composition or dosage form may contain from about 1 to 50 percent by weight an active ingredient to provide the optimum transdermal dosage. The optimum dosage is determined by pharmacodynamic and pharmacokinetics studies of the active ingredient. Such studies are described in Fundamentals of Clinical Pharmacokinetics by John Wagner, first edition, 1975, Drug Intelligence Publications Inc., Hamilton, Illinois and Pharmacokinetics, by Milo Gibaldi and Donald Perrier, First Edition, 1975, published by Marcel Dekker.

A preferred embodiment of the present invention is a transdermal device or drug delivery system having a sodium stearate-based matrix and a backing member which is impervious to the matrix and its contents. The active ingredient is diffusable through the matrix at a therapeutically effective constant rate when the device is affixed to the skin. The active ingredient is preferably dissolved or suspended in a nonaqueous liquid medium comprising the combination of propylene glycol and glycerine in a weight ratio of 35 percent to 65 percent.

It has been found that skin penetration profiles of an active ingredient, such as guanabenz, from non-polymeric matrices are equivalent to the penetration profiles from a saturated solution of propylene glycol and glycerine. While it is expected that the overall penetration would be similar regardless of whether propylene glycol or glycerine alone is used, surprisingly, it has been found that a combination of propylene glycol and glycerine in a weight ratio of about 35:65 shows a net increase of about 350 percent in overall penetration after the same period of time compared to matrices formed with either solvent alone.

It has been found that at particular ratios of propylene glycol and glycerine, the rate of penetration of some active ingredients through the skin is greatly enhanced. The liquid medium contains about 15 to 50, and, preferably about 35 percent by weight of propylene glycol, the remainder being glycerine. The presence of the sodium stearate in the matrix also tends to enhance penetration. The enhancement of the rate of penetration occurs with active ingredients which are soluble in the liquid medium to the amount of at least about 0.5 milligrams per millilitre preferably at least about 3 mg/ml, most preferably at least about 5 mg/ml. The pH is preferably about 8.5 to 9.5 with increased rate of penetration at the higher pH.

Surprisingly, it has been found that the matrix does not affect drug availability. It has been found that no retardation of drug penetration occurs when a drug-containing liquid medium is dispersed in a non-polymeric matrix. That is, the transdermal transfer rate of the drug is the same whether the drug-containing medium is a liquid containing only the combination of propylene glycol and glycerine in the indicated ratios or a semisolid including as well the gelling agent.

Also, it has been found that the combination of propylene glycol and glycerine as described heretofore may be further modified by the addition of other known penetration enhancers to achieve even greater penetration enhancement. Other known penetration enhancers include the aliphatic sulfoxides, such as dimethylsulfoxide, and azone.

Also, propylene glycol and glycerine may be combined in the herein defined limits with other compounds and compositions not known to be penetration enhancers, to provide greater penetration enhancement than the propylene glycol and glycerine alone. Such other compositions include ethyl alcohol and distilled water.

A transdermal dosage form of the present invention is preferably formed as follows. First the nonaqueous liquid medium containing propylene glycol and glycerine is heated to about 120° to 140°C. Simultaneously in a separate container sodium stearate dispersion is then added to the hot nonaqueous liquid medium. When all the sodium stearate is dissolved, the system is allowed to cool to a temperature compatible with the active ingredient. The active ingredient is either dissolved or suspended in the foregoing mixture at between 60 to 100°C. The composition is poured into suitable moulds and cooled to form a flexible matrix.

The cooled matrix is removed from the mould and, if desired, pressed into contact with an adhesive dispersed on an occlusive (that is, totally closed) backing of an impervious material, such as aluminium foil. The backing may have a flange area which extends around the outer periphery of the matrix. An adhesive may be applied to the flange area for attachment to the skin. A foil-backed

paper may be adhered to the flange and covers the face of the matrix as a release liner after manufacture and prior to use. As an alternative, a non-rate-controlling foraminous membrane may be applied over the face of the matrix and attached to the adhesive of the flanges.

The size of the dosage form, or patch, depends on the therapeutic dose per unit time and on the penetration rate of the active ingredient through the skin. A patch area of about 30 square centimetres ($cm^2$) is considered desirable. Therefore penetration rate is a major consideration.

Accordingly this invention also provides a patch suitable for transdermal administration of an active ingredient comprising a non-polymeric matrix containing from 10 to 93 percent by weight of a nonaqueous solvent medium comprising about 15 to 50 percent by weight of propylene glycol and about 50 to 85 percent by weight of glycerine the ratio of propylene glycol to glycerine being from about 1:1 to about 1:5 and a therapeutically effective amount of an active ingredient dissolved in said solvent medium. Additional active ingredient may be suspended in said solvent medium.

The rate of penetration and lag time are determined by studying _in vitro_ the penetration of the drug across cadaver skin, hairless mouse skin or other suitable membrane. A suitable apparatus is described by Y.W.Chien, P.R.Keshary, Y.C.Huang and P.P. Sarpotdar in "Comparative Controlled Skin Permeation of Nitroglycerin from Marketed Transdermal Delivery Systems", J.Pharmaceutical Sciences, Vol. 72, No.8, August 1983, pp. 968-970, the entire contents of which are incorporated herein by reference.

While the present invention has been described with regard to the use of a non-polymeric transdermal dosage form, it is to be understood that the principles of this invention can be applied to polymeric transdermal dosage forms. The ambit of the invention extends to all transdermal dosage forms in which the ingredients are compatible with a mixture of propylene glycol and glycerine.

The rate of penetration enhancement of the present invention due to the particular ratios of propylene glycol and glycerine is not limited to the presence of an alkaline metal stearate but may be achieved with other transdermal dosage form embodiments in which the combination of propylene glycol and glycerine may be dispersed in a carrier matrix. These include other non-polymeric matrices and porous polymeric membranes comprised of polyacrylates, polymethacrylates, polyvinyl alcohols, polyvinylpyrrolidone, silicones and the like.

A list of therapeutic classes and some specific examples of drugs suitable for incorporation into this invention is provided in Table A. The invention is not limited in application to guanabenz, and 17-beta-estradiol exemplified below, or solely to those drugs noted in Table A. The synergistic effect described is a drug independent interaction between the solvent medium and the skin barrier. This interaction in due course increases the permeability of the skin barrier and enhances the skin penetration of the drug.

Table A

| Therapeutic Category | Active Ingredient |
|---|---|
| Non-Steroidal Anti-inflammatory | Indomethacin |
| Beta-Blocker | Propranolol |
| Sedative/Anticonvulsant | Phenobarbital |
| Local Anaesthetic | Novocaine |
| | Benzocaine |
| | Xylocaine |
| Antifungal | Gentamycin |
| | Amphotericin ·B |
| Antihypertensive | Clonidine |
| | Guanabenz |
| Analgesic | Dezocine |
| Steroidal Contraceptives | Ethinyl Estradiol |
| | Levo-norgestrel |
| | 17-Beta-estradiol |
| Antiarrhythmic | Verapamil |

Penetration enhancement is achieved with those active ingredients which are soluble in the propylene glycol/glycerine solvent system, such as guanabenz and 17-beta estradiol. Some penetration enhancement was noted with levo-norgestrel which is relatively insoluble in the propylene glycol/glycerine solvent system when an additional solvent (ethanol) was added. The best results are obtained when the active ingredient is soluble in the solvent system to the extent of at least about three milligrams per millilitre.

The following non-limiting examples illustrate the the invention. In the examples, the steady rate of penetration, or flux, is indicated by the symbol: Jss. The cumulative amount of drug penetrated is indicated by the symbol: Q. The lag period to reach steady rate is indicated by the symbol: $t_L$. The standard deviation for statistical purposes is abbreviated: S.D. The standard error for statistical purposes is abbreviated: S.E. The abbreviation "g" denotes grams, "mg" denotes milligrams, "mcg" denotes micrograms and "ng" denotes nanograms.

## Example 1

This example illustrates the enhancement of penetration of an active ingredient using a mixture of propylene glycol and glycerine compared with either solvent alone.

The penetration of drug across cadaver skin was studied in vitro using a modified Franz diffusion cell apparatus as described by Y.E. Chien, P.R.Keshary, Y.C.Huang and P.P. Sarpotdar in "Comparative Controlled Skin Permeation of Nitroglycerin from Marketed Transdermal Delivery Systems," J. Pharmaceutical Sciences, Vol. 72, No. 8, August 1983, pp. 968-970. The formulations shown in the Table I-A were prepared.

### Table I-A

#### Guanabenz Formulations

| | 1A | 1B | 1C | 1D | 1E |
|---|---|---|---|---|---|
| Guanabenz free base | 10g | 2g | 10g | 2g | 6g |
| Propylene Glycol | 90g | - | 90g | - | 45g |
| Glycerine | - | 98g | | 98g | 45g |
| Sodium Stearate | - | - | 9.6g | 9.6g | 9.6g |
| Distilled Water | | | 5g | 5g | 5g |

Formulations 1A and 1B are simple saturated solutions of the drug in the respective solvents. The solutions were prepared by taking either 90g of propylene glycol or 98g of glycerine in a 150 ml beaker and dissolving either 10g or 2g of guanabenz free base in it under constant stirring. Thus the final weight of each solution was 100g. The amount of guanabenz used in each of the solvents was in excess. Thus in both examples guanabenz was partially suspended in the vehicle. These examples are used as controls to show the unique advantages of the invention.

Formulations 1C, 1D and 1E were made into matrices. To make the matrix the nonaqueous vehicle (propylene glycol/glycerine) was placed in a 150 ml beaker and heated to 125°C. Simultaneously in a 30 ml beaker sodium stearate was dispersed in the boiling distilled water. The sodium stearate slurry was then added to the nonaqueous vehicle slowly with constant stirring while maintaining the temperature at 125°C. When all of the sodium stearate was dissolved the system was allowed to cool under gentle mixing. The drug was added and dissolved in the hot solution when the temperature was between 60°C and 80°C. The translucent solution was poured into flat petri dishes. This enhanced the cooling process and formed an opaque, flexible matrix.

A measured portion of each of the formulations 1A-E was applied to the surface of the cadaver skin. The appearance of drug on the dermal side was monitored quantitatively over six days. The results are shown in Table I-B.

## Table I-B

Penetration (Q) in mcg/cm$^2$/hr $\pm$ S.E. of Guanabenz Formulations of Table I-A

| Formulation | 24 hrs. | 55 hrs. | 96 hrs. | 120 hrs. | 144 hrs. |
|---|---|---|---|---|---|
| 1A | 0.8$\pm$0.62 | 10.0$\pm$3.90 | 42.5$\pm$14.10 | 72.9$\pm$24.2 | 108.2$\pm$34.28 |
| 1B | 0.1$\pm$0.11 | 2.7$\pm$1.60 | 22.4$\pm$10.84 | 46.2$\pm$20.67 | 76.8$\pm$31.9 |
| 1C | 0.1$\pm$0.08 | 12.2$\pm$2.27 | 39 $\pm$ 3.27 | 65.1$\pm$ 3.44 | 95.4$\pm$ 7.67 |
| 1D | 0.8$\pm$0.75 | 5.6$\pm$4.61 | 27.2$\pm$12.19 | 48.6$\pm$16.07 | 77.8$\pm$22.35 |
| 1E | 0.0$\pm$0.0 | 12.7$\pm$2.98 | 81.7$\pm$22.72 | 180.3$\pm$46.17 | 294.5$\pm$78.05 |

The results show that the matrices formed according to formulations 1C and 1D provide skin penetration profiles that are statistically equivalent to the respective saturated solution of formulations 1A and 1B. This illustrates that the matrix does not affect drug availability.

The results show that a combination of propylene glycol and glycerine in a flexible matrix causes a net increase of about 350 percent in the overall penetration of the drug after six days, compared to matrices formed from either solvent alone. This illustrates the unexpected results of the present invention.

## Example 2

The example illustrates the synergistic effect of a propylene glycol and glycerine mixture on the transdermal penetration of an active ingredient in the absence of a matrix.

Following the procedure of Example 1 for formulations 1A & 1B saturated solutions were prepared substituting 17-beta-estradiol for guanabenz with propylene glycol alone, glycerine alone and a mixture of the two.

Table II-A shows the effect of solvent composition on the steady state rates of penetration and lag time of

AHP-8374

0165696

- 20 -

17-beta-estradiol across cadaver skin. The results show
that the steady rate of penetration of 17-beta-estradiol
is enhanced in a solvent system containing a 35:65 ratio
by weight of propylene glycol:glycerine. The results also
show that a corresponding decrease in lag time is achieved
with such a mixture.

## Table II-A

Effects of solvent composition on the steady state rates of
penetration and lag time of 17-beta-estradiol across
cadaver skin.

| Formulations | Solvent Composition | $t_L$ (hrs) | Jss ng/cm$^2$/hr | | Ratio of Enhancement |
|---|---|---|---|---|---|
| | | | Observed$\pm$S.E. | Corrected* | |
| 2A | Propylene glycol | 76.2$\pm$0.62 | 16.7$\pm$2.29 | 16.7 | 1.75 |
| 2B | 35:65 Propylene glycol/glycerine | 31.9$\pm$4.40 | 56.3$\pm$3.33 | 56.3 | 5.82 |
| 2C | 35:65 PG/G | 33.0$\pm$19.70 | 409$\pm$161 | 56.3 | 5.82 |
| 2D | Glycerine | 35.0$\pm$10.70 | 69$\pm$19.5 | 9.5 | 1 |

\*      Due to large differences in skin permeability between
specimens it is necessary to run a reference standard in
each transdermal experiment. The different rates of
penetration for the reference standard are equated via
an empirical proportionality constant. This constant is
used to "normalize" the experiments to one another.
Normalization is also used to compare results from
experiments with different samples of skin from different
species to account for high inter-species biological
variation.

It is to be noted that the rate of penetration of
17-beta-estradiol increased about 3 to 4 times when the
mixed solvent system was used compared to the use of the
pure solvents alone.

- 21 -

Table II-A shows the large interexperiment variation in the steady state flux of 17-beta-estradiol from mixtures of propylene glycol and glycerine. Using formulation 2B, four skin samples from the same person were used to determine the average flux. Using formulation 2C, skin samples from four different people were used to determine the average flux. Table II-B below illustrates the large intersubject variability that is responsible for the large standard error seen using formulation 2C in Table II-A. It is not uncommon in transdermal dosage form development to encounter ten fold intersubject variation in steady state flux and the patches now on the market are available in a range of surface areas to overcome this problem. It is also clear from Table II-B that the effect of the mixed solvent system is real in spite of the variability because each individual subject showed between a three and seven fold increase in cumulative amount of active ingredient penetration when the solvent was changed from pure glycerine to the mixture of propylene glycol and glycerine.

Table II-B

Variability in Seven Day Cumulative Flux in mcg/cm$^2$/hr

for 17-beta-estradiol formulations with different skins

| Subject Description | Solvent System | |
|---|---|---|
| | Glycerine | Propylene Glycol: Glycerine (35:65) |
| 60 yr.old,white male | 13 | 61 |
| 10 yr.old,white male | 5 | 18 |
| 70 yr.old,black female | 3 | 17 |
| 2 mo.old,white male | 16 | 114 |

- 22 -

## Example 3

This example illustrates the transdermal penetration enhancement of an active ingredient in a propylene glycol and glycerine solvent in conjunction with a known penetration enhancer. Azone and ethanol were used as the known penetration enhancers in separate formulations.

Formulations 3A to 3I were made into matrices. To make the matrix the nonaqueous vehicle (propylene glycol/ glycerine) (90g) was placed in a 150 ml beaker and heated to 125°C. Simultaneously in a 30 ml beaker sodium stearate (9.1g) was dispersed in a small amount of boiling distilled water (5g). The sodium stearate slurry was then added to the nonaqueous vehicle slowly with constant stirring while maintaining the temperature at 125°C (during which time substantially all of the water is boiled off). When all of the sodium stearate was dissolved the system was allowed to cool to 55-60°C under gentle mixing. To appropriate quantities (2-3g) of the hot gel solution guanabenz (.09-0.27g) and either the azone (.045-.135g) or ethanol (.45-1.35g) was added to and dissolved in the hot solution while the temperature was held between 60 and 80°C (preferably 60-65°C), to a total weight of 4.5 grams per test formulation. The translucent solution was poured into flat petri dishes. This enhanced the cooling process, and an opaque, flexible matrix was formed. The matrix patches contained 6.5 percent by weight of sodium stearate. These Guanabenz matrix formulations are shown in Table III-A.

Table III-A

Guanabenz formulations with additional penetration enhancer

| Formulation | Percent Guanabenz | Solvent Composition PG/Gly* | Azone % w/w | Ethanol % w/w |
|---|---|---|---|---|
| 3A | 2 | 20/80 | 1 | 30 |
| 3B | 2 | 20/80 | 2 | 30 |
| 3C | 2 | 20/80 | 3 | 30 |
| 3D | 5 | 35/65 | 2 | 10 |
| 3E | 5 | 35/65 | 2 | 20 |
| 3F | 5 | 35/65 | 2 | 30 |
| 3G | 5 | 35/65 | 1 | 30 |
| 3H | 5 | 35/65 | 3 | 30 |
| 3I | 6 | 50/50 | 3 | 30 |

*Propylene Glycol/Glycerine weight ratio to
each other in the solvent


Three levels of each of the three independent variables amount to a total of 27 possible combinations. Nine combinations were selected out of the possible 27 which were expected to give the best results and indicate a direction for further experimentation. These combinations are listed in Table III-A. Each experiment was normally run in quadruplicate and an internal reference was tested simultaneously. These results are listed in Table III-B.

## Table III-B

### Penetration rates and lag times for
### Guanabenz Formulations of Table III-A

| Form | No.of Replicates | $J_{ss}\pm$ S.D. $mcg/cm^2/hr$ | Rate Ratio $J_{ss}/2.4$ | $t_L \pm$ S.D. in hours | Lag Time Ratio $t_L/16$ |
|------|------------------|---------------------------------|-------------------------|--------------------------|--------------------------|
| 3A | 2* | 2.84±0.120 | 1.18 | 35±5.0 | 2.19 |
| 3B | 2* | 2.73±0.523 | 1.14 | 19±2.8 | 1.19 |
| 3C | 3* | 2.74±0.216 | 1.14 | 14±12.0 | 0.88 |
| 3D | 4 | 4.59±0.744 | 1.91 | 36±5.1 | 2.25 |
| 3E | 4 | 5.39±0.685 | 2.25 | 33±3.1 | 2.06 |
| 3F | 4 | 4.68±0.859 | 1.95 | 32±5.3 | 2.0 |
| 3G | 4 | 2.32±1.120 | 0.97 | 46±10.2 | 2.88 |
| 3H | 4 | 4.66±0.693 | 1.94 | 31±5.1 | 1.94 |
| 3I | 4 | 2.4 ±0.41 | 1 | 16±10.8 | 1 |

*Replicates either lost during experiment or rejected after statistical analysis.

The two "ratio" columns were created by arbitrarily choosing the last formula in the table as the standard. Then the observed average rates for each formula were divided by the rate observed for the standard. An analogous calculation was performed with the lag times. From the rate ratio column it can be seen that the best formula (3E) gave a more than two fold increase in rate of penetration over the standard. When lag time is considered, almost a three fold difference is seen between the standard and the formula (3G) with the longest lag time. Repetition of the experimentation will lead to the same or greater ratios. The absolute values may be different due to variations in skin samples.

Experiment 3F using 35:65 weight ratio of propylene glycol and glycerine shows enhanced penetration over Experiment 3B using 20:80 weight ratio of propylene glycol and glycerine at constant azone at 2% by weight. Comparing experiment 3C and 3H also shows such enhancement at constant

AHP-8374

0165696

- 25 -

azone of 3% by weight. Comparing 3B and 3G shows that there is less enhancement when azone is less than 2 percent.

Similarly, it is also shown in Experiments 3A, 3B and 3C, where azone varied from 1% to 2% to 3%, that there was no effect on penetration when the propylene glycol/glycerine ratio and ethanol concentration were constant.

The results in Table III-B show the effect of the ratio of propylene glycol to glycerine on penetration of guanabenz at a constant concentration of azone and ethanol. Similarly the results show the effect of azone concentration on guanabenz penetration at three fixed ratios of propylene glycol: glycerine in the vehicle and three levels of ethanol. The results show that there is a significant increase in penetration when azone concentration is increased from 1 to 2% . This simultaneously reduces lag period also. However, an increase from 2 to 3% azone concentration results in no increase in penetration.

The results also show the effect of ethanol concentration on the penetration of guanabenz at constant ratios of propylene glycol/glycerine in the vehicle. The results show that the greatest rate of penetration is achieved when starting ethanol concentration is 20%. Ethanol concentrations in excess of thirty percent (30%) cause the patch to be soft. The results show that ethanol does not affect lag time.

## Example 4

This example illustrates the penetration of an active ingredient (levonorgestrel) from various patch formulations using the Chien et al. in vitro skin penetration protocol referred to above. Four different formulations were studied.

Formulations 4A to 4D were made into matrices. To make the matrix the nonaqueous vehicle (propylene glycol/glycerine) (90g) was placed in a 150 ml beaker and heated to 125°C. Simultaneously in a 30 ml beaker sodium stearate (9.1g) was dispersed in the boiling distilled water. The sodium stearate slurry was then added to the nonaqueous vehicle slowly with constant stirring while maintaining the temperature at 125°C. When all of the sodium stearate was dissolved, the system was allowed to cool to 55-60°C under gentle mixing. The levo-norgestrel (.0045g) and either the azone (.135g) or ethanol (0.9g) was added to and dissolved in 2-3g of the hot gel solution while the temperature was held between 60°C and 80°C (preferably 60-65°C), to make a total of 4.5 grams. The translucent solution was poured into flat petri dishes. This enhanced the cooling process, and an opaque, flexible matrix was formed. The matrix patches contained 6.5 percent by weight of sodium stearate. The levo-norgestrel formulations are shown in Table IV-A, and the results are shown in Table IV-B.

## Table IV-A

### Formulations with levo-Norgestrel

| Form. | PG/Gly Ratio | Ethanol % w/w | Azone % w/w |
|-------|--------------|---------------|-------------|
| 4A    | 35/65        | --            | --          |
| 4B    | 35/65        | 20            | --          |
| 4C    | 35/65        | --            | 3           |
| 4D    | 35/65        | 20            | 3           |

Table IV-B

Penetration rates and lag times of the

levo-norgestrel formulations of Table IV-A

| Form. | Jss rate (S.E.) of penetration $mcg/cm^2/day$ | $t_L$ (S.E.) hrs. |
|-------|-----------------------------------------------|-------------------|
| 4A | 0.39 (0.10) | 73 (0.82) |
| 4B | 1.67 (0.69) | 63 (6.5) |
| 4C | 1.25 (0.22) | 56 (8.5) |
| 4D | 1.68 (0.33) | 39 (10) |

The steady state rates of penetration are listed in Table IV-B. The results show that formulae 4B and 4D are superior to the rest of the formulas. Formula 4D appears to be the best of the four due to shorter lag periods. However, the same rates of penetration can be achieved in the absence of azone.

Example 5

This example illustrates the transdermal penetration enhancement of an active ingredient in a propylene glycol and glycerine solvent in conjunction with azone and with ethanol.

Following the procedure of Example 3 transdermal dosage forms were prepared substituting 17-beta-estradiol for guanabenz in a sodium stearate matrix that included a mixture of propylene glycol (PG) and glycerine(Gly), and the same with either azone or ethanol. All formulations were gelled using 6.5 percent by weight of sodium stearate. The formulations are shown in Table V-A.

- 28 -

## Table V-A

Effect of penetration enhancers on the penetration of 17-beta-estradiol through human cadaver skin in vitro

| Form. | Solvent Composition | Jss±S.E. mcg/ cm$^2$/hr | $t_L$±S.E. hours |
|---|---|---|---|
| 5A | 35/65:PG/Gly | 0.132±0.0078 | 40± 3.5 |
| 5B | 35/65:PG/Gly+20%EtOH USP | 0.209±0.276 | 27±6.0 |
| 5C | 35/65:PG/Gly+3% Azone | 0.225±0.0142 | 44±3.3 |

Table V-A shows that the addition of either 20 percent by weight of ethanol USP or 3 percent by weight of azone to the formulation increases the rate of penetration of 17-beta-estradiol significantly.

## Example 6

This example illustrates the penetration of levo-norgestrel from saturated solutions of pure propylene glycol, pure glycerine and a mixture containing equal parts of propylene glycol and glycerine.

The saturated solutions were prepared according to the procedure of Example 1 for formulations 1A and 1B but substituting levo-norgestrel for guanabenz. The formulations and results are given in Table VI-A.

## Table VI-A
### levo-Norgestrel Penetration

| Formulation | Solvent | Observed Jss ± S.E. (mcg/cm$^2$/hr) | $t_L$ ± S.E. hours |
|---|---|---|---|
| 6A | Pure PG | 0.027±0.0057 | 57± 9.2 |
| 6B | 50/50 PG/Gly | 0.02 ±0.0021 | 60± 2.3 |
| 6C | Pure Glycerine | 0.0089±0.00072 | 71± 0.7 |

This example read in conjunction with the other examples illustrates the criticality of active ingredient solubility in the propylene glycol and glycerine solvent system to the penetration enhancement in the transdermal dosage form. No enhancement of the rate of transdermal penetration of levo-norgestrel is shown in Table VI-A. Table VII below shows that levo-norgestrel has no detectable solubility in the propylene glycol and glycerine solvent.

## Example 7

This example illustrates the solubility of various active ingredients in propylene glycol or glycerine alone and in mixtures thereof.

Procedure

Excess of active ingredient is added to the solvent and put on a shaker bath which is maintained at constant temperature for a minimum of 96 hours and then filtered through a Millipore filter of porosity 0.2 microns while hot. An exact amount of saturated solution is withdrawn into a volumetric flask while maintaining temperature. The saturated solution is diluted in suitable solvent and the content of active ingredient measured by high pressure liquid chromatography or other suitable analytical method.

The results are shown in Table VII.

### Table VII

Equilibrium solubility of active ingredients in solvents in mg/ml (temperature°C)

| Active Ingredient | Propylene Glycol | 50/50PG/Gly | 35/65PG/Gly | Glycerine |
|---|---|---|---|---|
| 17-beta-estradiol | 51.21 (37) | | 7.0 (32) | 2.16(37) |
| Guanabenz | 47.0 (34) | 29.4(34) | 21.18(34) | 7.97(34) |
| Levo-norgestrel | 2.51 (37) | | non detected (32) | 0.89(37) |

The solubility data in Table VII taken together with the data in the other examples indicates the critical role of solubility in determining the magnitude of penetration enhancement provided by various combinations of propylene glycol and glycerine.

## Example 8

This example illustrates the penetration enhancement effect of the subject propylene glycol and glycerine combination when present in the drug delivery system at a weight concentration of only 10 percent. The example further illustrates that the presence of water in the system, even in a substantial amount (41.25 percent), does not affect the enhancement of the rate of penetration provided by the subject propylene glycol/glycerine combination. Still further, this example illustrates that the rate of penetration of levonorgestrel is in fact enhanced by the subject propylene glycol/glycerine combination. Thus, even though the solubility of levonorgestrel in the propylene glycol/glycerine/alcohol/ water solvent medium was only about .5 mg/ml. (instead of the preferred level of at least 3 mg/ml), a 2½ fold increase in the rate of penetration was observed for the propylene glycol/glycerine system (8B).

The penetration of drug across cadaver skin was studied _in vitro_ using a modified Franz diffusion cell apparatus as described in Chien et al., cited in Example 1. Solution 8A was prepared by adding 1.0 grams of levonorgestrel to 99 grams of a premade 50/50 (by weight) mixture of alcohol USP and distilled water. 8B is a gel matrix and was made substantially as Example 1E was made. In this case, however, the propylene glycol/glycerine mixture was first added to the premade 50/50 alcohol USP/water mixture. The resulting mixture was then heated to only 60-65° C and the sodium stearate was then added. While maintaining the mixture at these temperatures the drug (levonorgestrel) was then added.

A measured portion of each formulation, 8A & B, was applied to the surface of the cadaver skin. The appearance of drug on the dermal side was monitored quantitatively over six days. The results are shown in Table VIII.

### Table VIII

#### Soluble levo-norgestrel formulations and their rates of penetration

| Ingredient | 8A | 8B |
|---|---|---|
| Levonorgestrel | 1.0 | 1.0 |
| Sodium Stearate | - | 6.5 |
| 35/65 PG/G | - | 10.0 |
| 50/50 Alcohol USP/Water | 99.0 | 82.5 |
| | 100.0 | 100.0 |
| Flux $\pm$ S.E. ($mcg/cm^2/hr$) | $0.181\pm0.0134$ | $0.489\pm0.098$ |

These results show that the rate of penetration of levonorgestrel is enhanced significantly (2.7 times) by as little as 10 weight percent of the inventive combination of propylene glycol and glycerine.

### Example 9

In this example Guanabenz formulations containing 10-40 percent of the inventinve combination of propylene glycol and glycerine in a 35:65 weight ratio were made. In these formulations, alcohol and water were present in amounts varying from 0-40 percent and 40-80 percent, respectively. All of the formulations contained 2 percent azone, 6.5 percent sodium stearate and 3.0 percent of the active ingredient, Guanabenz.

Batches were prepared containing 4.5 grams each of the particular formulation. They were prepared by first mixing Guanabenz free base (0.135g), azone (0.09g), propylene glycol/glycerin (35:65) (0.45-1.8g), water (1.77-3.53g) and sodium stearate (0.29g). The mixture was then heated to about 60°C until the sodium stearate completely dissolved. Then alcohol USP (0-1.77g) was added while mixing. The resulting translucent formulation was poured into a flat Petri dish and cooled to form an opaque flexible matrix.

The rates of penetration (flux or Jss values) in this experiment were measured using hairless mouse skin (from mice 5 to 8 weeks old) over a 1 day period. The formulations and flux Jss values are given in Table IX below.

Table IX

| Formulation | PG/G %w/w | Ethanol % w/w | Water % w/w | Jss $(mcg/cm^2/m)$ + S.E. |
|---|---|---|---|---|
| 9A | 10.0 | 39.25 | 39.25 | 12.806+1.756 |
| 9B | 10.0 | 29.25 | 49.25 | 11.695+0.976 |
| 9C | 20.0 | 29.25 | 39.25 | 16.095+1.252 |
| 9D | 10.0 | 19.25 | 59.25 | 6.889 +0.910 |
| 9E | 20.0 | 19.25 | 49.25 | 20.980+4.508 |
| 9F | 30.0 | 19.25 | 39.25 | 13.935+2.728 |
| 9G | 10.0 | 9.25 | 69.25 | 11.976+0.704 |
| 9H | 20.0 | 9.25 | 59.25 | 15.117+3.218 |
| 9I | 30.0 | 9.25 | 49.25 | 12.409+1.761 |
| 9J | 40.0 | 9.25 | 39.25 | 12.681+2.328 |
| 9K | 10.0 | 0.0 | 78.50 | 9.156+0.315 |

These results show that the penetration enhancement of a propylene glycol/glycerin solvent system is the highest when the 35:65 propylene glycol/glycerin combination is present at 20 percent of the total weight of the patch. This improvement was seen at three different concentration ratios of alcohol and water (viz. 30:40, 20:50 and 10:60). This example also shows that varying the amounts of water and alcohol did not adversely affect the penetration enhancement. Thus, the 20 percent propylene glycol/glycerin combination gave the highest rates of penetration at three different ratios of alcohol and water. Conversely, the lowest rate of penetration was shown at 10 percent propylene glycol/glycerin combination in 4 out of 5 cases. In the fifth case, the rate was substantially the same as that for a 40 percent propylene glycol/glycerin formulation. Example 1 above (run on human cadaver skin) showed at least a threefold enhancement of the rate of penetration of the inventive propylene glycol/glycerin combination over formulations containing only either propylene glycol or glycerin.

CLAIMS

1. A pharmaceutical composition for the transdermal delivery of a therapeutic agent dispersed in a solvent system characterised in that the solvent system comprises a mixture of propylene glycol and glycerine in the ratio of about 1:1 to about 1:5 by weight of each other and if required such other pharmaceutically acceptable solvent or solvents necessary to achieve a solubility of at least 0.5 milligrams/millilitre of the therapeutic agent in the solvent system, the amount of propylene glycol and glycerine being such as to enhance the transdermal penetration of the therapeutic agent.

2. A composition as claimed in Claim 1 in which the propylene glycol-glycerine mixture comprises 10 to 93 per cent by weight of the composition.

3. A composition as claimed in Claim 1 or Claim 2 in which the propylene glycol:glycerine ratio is about 1:1 to about 1:4.

4. A composition as claimed in any one of Claims 1 to 3 further comprising an additional penetration enhancer.

5. A composition as claimed in any one of Claims 1 to 4 wherein the therapeutic agent is soluble in the solvent system in an amount of at least 3 milligrams per millilitre of solvent.

6. A composition as claimed in any one of Claims 1 to 5 wherein the therapeutic agent comprises 1 to 50 per cent of the composition.

7.      A pharmaceutical transdermal dosage form
        comprising a matrix having dispersed therein a
        composition as claimed in any one of Claims
        1 to 6.

8.      A dosage form as claimed in Claim 7 in which
        the matrix is a non-polymeric gel.

9.      A dosage form as claimed in Claim 8 in which
        the non-polymeric gel comprises a metal
        salt of a fatty acid.

10.     A pharmaceutical dosage form for transdermal
        administration of an active ingredient comprising
        a thin flexible non-polymeric matrix in planar
        form further comprising:
            a) about 3 to 50 per cent by weight of
               solid non-polymeric gel;
            b) about 10 to 93 per cent by weight of a
               solvent medium dispersed in said gel
               comprising propylene glycol and glycerine
               in a ratio of about 35:65 by weight of
               each other; and
            c) from 1 to 50 per cent by weight of a
               therapeutic agent dispersed in said
               solvent medium.

11.     A dosage form as claimed in Claim 9 or Claim 10
        in which the non-polymeric matrix comprises a
        gelling agent selected from sodium stearate, sodium
        caprylate, sodium laurate, sodium myristate, sodium
        palmilate, sodium oleate, potassium caprylate,
        potassium laurate, potassium myristate, potassium
        palmitate, potassium oleate and potassium stearate.

12. A dosage form as claimed in any one of Claims 7 to 11 in which the matrix comprises about 6 per cent by weight of the dosage form.

13. A drug delivery system suitable for the transdermal administration of a therapeutic agent comprising a dosage form as claimed in any one of claims 7 to 12 and an impervious backing member adapted to attach said system to the skin.

14. A dosage form or composition as claimed in any one of Claims 1 to 14 in which the therapeutic agent is guanabenz, 17-beta-estradiol, indomethacin, propanolol, phenobarbital, novacaine, benzocaine, xylocaine, gentamycin, amphotericim, clonidine, dezocine, ethinyl estradiol, levonorgestrel or verapamil.

15. A process for preparing a transdermal pharmaceutical composition which comprises bringing into association a transdermally effective therapeutic agent, propylene glycol, glycerine and if required such other solvent or solvents to achieve a solubility of at least about 0.5 milligrams per millilitre of the therapeutic agent in the mixture of solvents, the ratio of propylene glycol to glycerine being from about 1:1 to 1:5 by weight of each other and the amount of propylene glycol - glycerine mixture being such as to enhance the penetration of the drug.

16.     A process for preparing a pharmaceutical dosage
        form which comprises bringing into association
        a matrix or matrix forming agent, a transdermally
        effective therapeutic agent, propylene glycol,
        other solvent or solvents to achieve a
        solubility of at least 0.5 milligrams per
        millilitre of the therapeutic agent in the
        mixture of solvents; the ratio of propylene
        glycol to glycerine being from about 1:1 to 1:5
        by weight of each other and the amount of
        propylene glycol - glycerine mixture being such
        as to enhance the penetration of the drug, and
        if required forming the matrix from the matrix
        forming agent at any suitable stage.

17.     A process as claimed in Claim 16 in which the
        matrix comprises a non polymeric gel.

18.     A process as claimed in Claim 17 in which the
        matrix comprises a metal salt of a fatty acid.